# EUROPEAN PATENT SPECIFICATION

(11) **EP 4 090 662 B1**
(45) Date of publication and mention of the grant of the patent: **12.06.2024**
(21) Application number: 21701689.8
(22) Date of filing: 14.01.2021
(51) Int. Cl.: C07D 489/02

(54) **PREPARATION OF BUPRENORPHINE**
ZUBEREITUNG VON BUPRENORPHIN
PRÉPARATION DE BUPRÉNORPHINE

(30) Priority: 17.01.2020 US 202062962335 P
(43) Date of publication of application: 23.11.2022
(73) Proprietor: River Stone Biotech ApS, 2100 Copenhagen (DK)
(72) Inventor: SANTELLA, Marco, 2100 Copenhagen (DK)
(74) Representative: Birkeland, Morten
(86) International application number: PCT/EP2021/050692
(87) International publication number: WO 2021/144362

(56) References cited:
- WO-A1-2018/211331
- MARTON J ET AL: "Herstellung von 6,14-Ethenomorphinan-Derivaten", LIEBIGS ANNALEN DER CHEMIE, VERLAG CHEMIE GMBH. WEINHEIM, DE, 1 January 1993 (1993-01-01), pages 915-919, XP002519987, ISSN: 0170-2041, DOI: 10.1002/JLAC.1993199301144
- ATTILA SIPOS ET AL: "First Synthesis and Utilization of Oripavidine - A Concise and Efficient Route to Important Morphinans and Apomorphines", HELVETICA CHIMICA ACTA, vol. 92, no. 7, 1 July 2009 (2009-07-01), pages 1359-1365, XP055638683, ISSN: 0018-019X, DOI: 10.1002/hlca.200800438 cited in the application

## Description

### BACKGROUND OF THE DISCLOSURE

### Field of the Disclosure

This disclosure relates to methods for preparing buprenorphine. More particularly, the present disclosure relates to methods for preparing buprenorphine from nororipavine.

### Description of Related Art

Total synthesis of natural opiate compounds or semisynthetic opioids is complex and not commercially competitive (Rinner et al., Top. Curr. Chem. 309:33-66 (2012)). While natural opiates are obtained from plants, opioids are often obtained semi-synthetically using natural opiate precursors. Buprenorphine and other semisynthetic opioids are, or can be, made from thebaine, an opiate alkaloid (Hudlicky, Can. J. Chem. 93(5):492-501 (2015)). Thebaine is currently obtained by crop cultivation of and extraction from plants of the *Papaver* genus. Several possible methods to prepare buprenorphine have been reported from thebaine. A known and likely commercial route to buprenorphine is made up of 6 major steps, starting from thebaine. (Machara et al., Adv. Synth. Catal. 354(4):613-26 (2012); Werner et al., J. Org. Chem. 76(11):4628-34 (2011)). The first 3 steps are a Diels-Alder reaction of thebaine with methyl vinyl ketone to form a 4+2 product, hydrogenation of the carbon-carbon double bond of the resultant product, and addition of a tertiary butyl group via a Grignard reaction. The final steps are N- and O- demethylation and cyclopropyl alkylation. The number of steps can increase to 8, if the N- and O-demethylation and N-alkylation steps are performed in 2 stages, rather than 1. The order of the hydrogenation and Grignard steps may be reversed but most, if not all, economically viable preparations include the 3 above-mentioned steps prior to the N-demethylation step. One challenge of this known preparation of buprenorphine is the exchange of the N-methyl group for an N-cyclopropyl group. N-demethylation methods can involve highly toxic reagents such as cyanogen bromide (von Braun, J. Chem. Ber., 33:1438-1452 (1900)) and chloroformate reagents (Cooley et al., Synthesis, 1:1-7 (1989); Olofson et al., J. Org. Chem., 49:2081-2082 (1984)) or may proceed in low yield, for example, by producing N-oxide intermediates (Polonovski reaction: Kok et al., Adv Synth. Catal., 351:283-286 (2009); Dong et al., J. Org. Chem., 72:9881-9885 (2007)). These methods generate significant amounts of toxic waste. The harsh conditions used for demethylation (*e.g.,* strong bases and high temperatures) generate a significant amount of impurities, requiring additional purification and lowering yields. Attempts to reduce impurities and improve yields have been made by avoiding the O-demethylation step, by using oripavine as starting material, but a principal obstacle to an efficient synthesis remains the N-demethylation step.

Accordingly, there remains a need for an improved route to buprenorphine, such as a route that is shorter, more efficient (due to, e.g., improved total yield, decreased impurities), and/or produces less toxic waste.

### SUMMARY OF THE DISCLOSURE

One aspect of the disclosure relates to a method of preparing buprenorphine, or a salt thereof, from Compound HO-I-H, or a salt thereof: 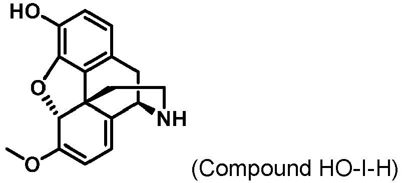 comprising:
(i)(F) in a first solvent system S1 comprising a polar protic solvent, reacting Compound HO-I-H with benzyl halide, benzyl sulfonate, or activated benzyl alcohol to provide Compound BnO-I-Bn: 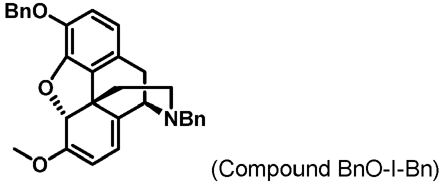
(ii)(B) in a second solvent system S2 comprising a polar protic solvent, reacting Compound BnO-I-Bn with methyl vinyl ketone to provide Compound BnO-II-Bn: 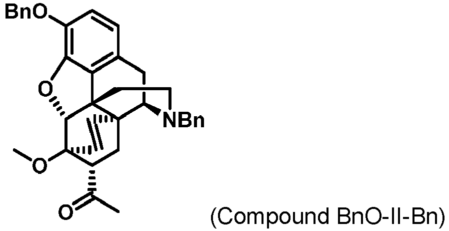
(iii)(D) in a third solvent system S3 comprising a nonpolar solvent, reacting Compound BnO-II-Bn with a *tert*-butylmagnesium compound to provide Compound BnO-IIIA-Bn: 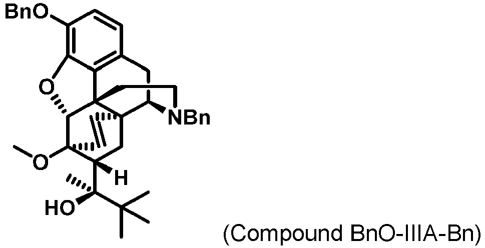
(iv)(C) reacting Compound BnO-IIIA-Bn with H₂ in the presence of a hydrogenation catalyst to provide Compound HO-IV-H. 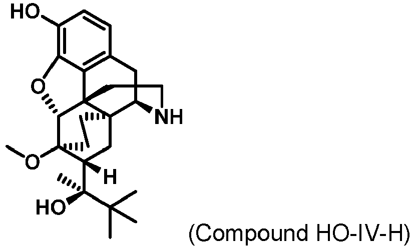
(v)(A1) reacting Compound HO-IV-H with cyclopropane carboxaldehyde followed by a hydride source; or
(v)(A2) reacting Compound HO-IV-H with cyclopropanecarboxylic acid halide followed by a reducing agent; or
(v)(A3) reacting Compound HO-IV-H with cyclopropylmethyl halide or activated cyclopropane methanol;
to provide buprenorphine.

Aspects and embodiments of the disclosure related to methods of preparing buprenorphine from Compound HO-I-H provide improved routes to buprenorphine that can be shorter, more efficient, and/or produce less toxic waste than, e.g., current commercial routes to buprenorphine. As a result, these aspects and embodiments can be well-suited for commercial (e.g., kg-scale) production of buprenorphine. Further, in certain aspects and embodiments, the synthetic routes disclosed herein advantageously avoid the harsh conditions and/or toxic byproducts of an N-demethylation step and can accordingly be particularly well-suited for producing buprenorphine on a commercial, e.g., kg, scale.

### DETAILED DESCRIPTION

The present invention is defined by the appended claims. Any embodiment not falling within the scope of the appended claims does not form part of the invention.

The particulars shown herein are by way of example and for purposes of illustrative discussion of the preferred embodiments of the invention only and are presented in the cause of providing what is believed to be the most useful and readily understood description of the principles and conceptual aspects of various embodiments of the invention. In this regard, no attempt is made to show structural details of the invention in more detail than is necessary for the fundamental understanding of the invention, the description taken with the drawings and/or examples making apparent to those skilled in the art how the several forms of the invention may be embodied in practice. Thus, before the disclosed processes and devices are described, it is to be understood that the aspects described herein are not limited to specific embodiments, apparati, or configurations, and as such can, of course, vary. It is also to be understood that the terminology used herein is for the purpose of describing particular aspects only and, unless specifically defined herein, is not intended to be limiting.

The terms "a," "an," "the" and similar referents used in the context of describing the invention (especially in the context of the following claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it were individually recited herein. Ranges can be expressed herein as from "about" one particular value, and/or to "about" another particular value. When such a range is expressed, another aspect includes from the one particular value and/or to the other particular value. Similarly, when values are expressed as approximations, by use of the antecedent "about," it will be understood that the particular value forms another aspect. It will be further understood that the endpoints of each of the ranges are significant both in relation to the other endpoint, and independently of the other endpoint.

All methods described herein can be performed in any suitable order of steps unless otherwise indicated herein or otherwise clearly contradicted by context. The use of any and all examples, or exemplary language (e.g., "such as") provided herein is intended merely to better illuminate the invention and does not pose a limitation on the scope of the invention otherwise claimed. No language in the specification should be construed as indicating any non-claimed element essential to the practice of the invention.

Unless the context clearly requires otherwise, throughout the description and the claims, the words 'comprise', 'comprising', and the like are to be construed in an inclusive sense as opposed to an exclusive or exhaustive sense; that is to say, in the sense of "including, but not limited to". Words using the singular or plural number also include the plural and singular number, respectively. Additionally, the words "herein," "above," and "below" and words of similar import, when used in this application, shall refer to this application as a whole and not to any particular portions of the application.

As will be understood by one of ordinary skill in the art, each embodiment disclosed herein can comprise, consist essentially of or consist of its particular stated element, step, ingredient or component.

Unless otherwise indicated, all numbers expressing quantities of ingredients, properties such as molecular weight, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should at least be construed in light of the number of reported significant digits and by applying ordinary rounding techniques. When further clarity is required, the term "about" has the meaning reasonably ascribed to it by a person skilled in the art when used in conjunction with a stated numerical value or range.

All percentages, ratios and proportions herein are by weight, unless otherwise specified. A weight percent (weight %, also as wt. %) of a component, unless specifically stated to the contrary, is based on the total weight of the composition in which the component is included (e.g., on the total amount of the reaction mixture).

Each group member may be referred to and claimed individually or in any combination with other members of the group or other elements found herein. It is anticipated that one or more members of a group may be included in, or deleted from, a group for reasons of convenience and/or patentability. When any such inclusion or deletion occurs, the specification is deemed to contain the group as modified thus fulfilling the written description of all Markush groups used in the appended claims.

Several embodiments of this invention are described herein. Of course, variations on these described embodiments will become apparent to those of ordinary skill in the art upon reading the foregoing description. The inventor expects skilled artisans to employ such variations as appropriate, and the inventors intend for the invention to be practiced otherwise than specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

Terms used herein may be preceded and/or followed by a single dash, "-", or a double dash, "=", to indicate the bond order of the bond between the named substituent and its parent moiety; a single dash indicates a single bond and a double dash indicates a double bond or a pair of single bonds in the case of a spiro-substituent. In the absence of a single or double dash it is understood that a single bond is formed between the substituent and its parent moiety; further, substituents are intended to be read "left to right" with reference to the chemical structure referred to unless a dash indicates otherwise. For example, arylalkyl, arylalkyl-, and -alkylaryl indicate the same functionality.

For simplicity, chemical moieties are defined and referred to throughout primarily as univalent chemical moieties (*e.g.,* alkyl, aryl, etc.). Nevertheless, such terms are also used to convey corresponding multivalent moieties under the appropriate structural circumstances clear to those skilled in the art. For example, while an "alkyl" moiety can refer to a monovalent radical (*e.g.* CH₃-CH₂-), in some circumstances a bivalent linking moiety can be "alkyl," in which case those skilled in the art will understand the alkyl to be a divalent radical (*e.g.,* -CH₂-CH₂-), which is equivalent to the term "alkylene." (Similarly, in circumstances in which a divalent moiety is required and is stated as being "aryl," those skilled in the art will understand that the term "aryl" refers to the corresponding divalent moiety, arylene). All atoms are understood to have their normal number of valences for bond formation (*i.e.,* 4 for carbon, 3 for N, 2 for O, and 2, 4, or 6 for S, depending on the oxidation state of the S). Nitrogens in the presently disclosed compounds can be hypervalent, e.g., an *N*-oxide or tetrasubstituted ammonium salt. On occasion a moiety may be defined, for example, as -B-(A)ₐ, wherein a is 0 or 1. In such instances, when a is 0 the moiety is -B and when a is 1 the moiety is -B-A.

As used herein, the term "alkyl" or "alkane" includes a saturated hydrocarbon having a designed number of carbon atoms, such as 1 to 40 carbons (i.e., inclusive of 1 and 40), 1 to 35 carbons, 1 to 25 carbons, 1 to 20 carbons, or 1, 2, 3, 4, 5, 6, 7, 8, 9, 10, 11, 12, 13, 14, 15, 16, 17, or 18. Alkyl groups or alkanes may be straight or branched and depending on context, may be a monovalent radical or a divalent radical (i.e., an alkylene group). For example, the moiety "-(C₁-C₆ alkyl)-O-" signifies connection of an oxygen through an alkylene bridge having from 1 to 6 carbons and C₁-C₃ alkyl represents methyl, ethyl, and propyl moieties. Examples of "alkyl" include, for example, methyl, ethyl, propyl, isopropyl, butyl, iso-, sec- and tert-butyl, pentyl, and hexyl. Examples of "alkane" include, for example, methane, ethane, propane, isopropane, butane, isobutane, sec-butane, tert-butane, pentane, hexane, heptane, and octane.

The term "alkoxy" represents an alkyl group of indicated number of carbon atoms attached to the parent molecular moiety through an oxygen bridge. Examples of "alkoxy" include, for example, methoxy, ethoxy, propoxy, and isopropoxy.

The term "alkenyl" as used herein, unsaturated hydrocarbon containing from 2 to 10 carbons (i.e., inclusive of 2 and 10), 2 to 8 carbons, 2 to 6 carbons, or 2, 3, 4, 5 or 6, unless otherwise specified, and containing at least one carbon-carbon double bond. Alkenyl group may be straight or branched and depending on context, may be a monovalent radical or a divalent radical (i.e., an alkenylene group). For example, the moiety "-(C₂-C₆ alkenyl)-O-" signifies connection of an oxygen through an alkenylene bridge having from 2 to 6 carbons. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, 3-decenyl, and 3,7-dimethylocta-2,6-dienyl.

The term "alkynyl" as used herein, unsaturated hydrocarbon containing from 2 to 10 carbons (i.e., inclusive of 2 and 10), 2 to 8 carbons, 2 to 6 carbons, or 2, 3, 4, 5 or 6 unless otherwise specified, and containing at least one carbon-carbon triple bond. Alkynyl group may be straight or branched and depending on context, may be a monovalent radical or a divalent radical (i.e., an alkynylene group). For example, the moiety "-(C₂-C₆ alkynyl)-O-" signifies connection of an oxygen through an alkynylene bridge having from 2 to 6 carbons. Representative examples of alkynyl include, but are not limited to, acetylenyl, 1-propynyl, 2-propynyl, 3-butynyl, 2-pentynyl, and 1-butynyl.

The term "aryl" represents an aromatic ring system having a single ring (e.g., phenyl) which is optionally fused to other aromatic hydrocarbon rings or non-aromatic hydrocarbon or heterocyclic rings. "Aryl" includes ring systems having multiple condensed rings and in which at least one is carbocyclic and aromatic, (e.g., 1,2,3,4-tetrahydronaphthyl, naphthyl). Examples of aryl groups include phenyl, 1-naphthyl, 2-naphthyl, indanyl, indenyl, dihydronaphthyl, fluorenyl, tetralinyl, and 6,7,8,9-tetrahydro-5*H*-benzo[a]cycloheptenyl. "Aryl" also includes ring systems having a first carbocyclic, aromatic ring fused to a nonaromatic heterocycle, for example, 1H-2,3-dihydrobenzofuranyl and tetrahydroisoquinolinyl. The aryl groups herein are unsubstituted or, when specified as "optionally substituted", can unless stated otherwise be substituted in one or more substitutable positions with various groups as indicated.

The term "heteroaryl" refers to an aromatic ring system containing at least one aromatic heteroatom selected from nitrogen, oxygen and sulfur in an aromatic ring. Most commonly, the heteroaryl groups will have 1, 2, 3, or 4 heteroatoms. The heteroaryl may be fused to one or more non-aromatic rings, for example, cycloalkyl or heterocycloalkyl rings, wherein the cycloalkyl and heterocycloalkyl rings are described herein. In one embodiment of the present compounds the heteroaryl group is bonded to the remainder of the structure through an atom in a heteroaryl group aromatic ring. In another embodiment, the heteroaryl group is bonded to the remainder of the structure through a non-aromatic ring atom. Examples of heteroaryl groups include, for example, pyridyl, pyrimidinyl, quinolinyl, benzothienyl, indolyl, indolinyl, pyridazinyl, pyrazinyl, isoindolyl, isoquinolyl, quinazolinyl, quinoxalinyl, phthalazinyl, imidazolyl, isoxazolyl, pyrazolyl, oxazolyl, thiazolyl, indolizinyl, indazolyl, benzothiazolyl, benzimidazolyl, benzofuranyl, furanyl, thienyl, pyrrolyl, oxadiazolyl, thiadiazolyl, benzo[1,4]oxazinyl, triazolyl, tetrazolyl, isothiazolyl, naphthyridinyl, isochromanyl, chromanyl, isoindolinyl, isobenzothienyl, benzoxazolyl, pyridopyridinyl, purinyl, benzodioxolyl, triazinyl, pteridinyl, benzothiazolyl, imidazopyridinyl, imidazothiazolyl, benzisoxazinyl, benzoxazinyl, benzopyranyl, benzothiopyranyl, chromonyl, chromanonyl, pyridinyl-*N*-oxide, isoindolinonyl, benzodioxanyl, benzoxazolinonyl, pyrrolyl *N*-oxide, pyrimidinyl *N*-oxide, pyridazinyl *N*-oxide, pyrazinyl *N*-oxide, quinolinyl *N*-oxide, indolyl *N*-oxide, indolinyl *N*-oxide, isoquinolyl *N*-oxide, quinazolinyl *N*-oxide, quinoxalinyl *N*-oxide, phthalazinyl *N*-oxide, imidazolyl *N*-oxide, isoxazolyl *N*-oxide, oxazolyl *N*-oxide, thiazolyl *N*-oxide, indolizinyl *N*-oxide, indazolyl *N*-oxide, benzothiazolyl *N*-oxide, benzimidazolyl *N*-oxide, pyrrolyl *N*-oxide, oxadiazolyl *N*-oxide, thiadiazolyl *N*-oxide, triazolyl *N*-oxide, tetrazolyl *N*-oxide, benzothiopyranyl S-oxide, benzothiopyranyl S,S-dioxide. Preferred heteroaryl groups include pyridyl, pyrimidyl, quinolinyl, indolyl, pyrrolyl, furanyl, thienyl and imidazolyl, pyrazolyl, indazolyl, thiazolyl and benzothiazolyl. In certain embodiments, each heteroaryl is selected from pyridyl, pyrimidinyl, pyridazinyl, pyrazinyl, imidazolyl, isoxazolyl, pyrazolyl, oxazolyl, thiazolyl, furanyl, thienyl, pyrrolyl, oxadiazolyl, thiadiazolyl, triazolyl, tetrazolyl, isothiazolyl, pyridinyl-*N*-oxide, pyrrolyl *N-*oxide, pyrimidinyl *N*-oxide, pyridazinyl *N*-oxide, pyrazinyl *N*-oxide, imidazolyl *N*-oxide, isoxazolyl *N*-oxide, oxazolyl *N*-oxide, thiazolyl *N*-oxide, pyrrolyl *N*-oxide, oxadiazolyl *N*-oxide, thiadiazolyl *N*-oxide, triazolyl *N*-oxide, and tetrazolyl *N*-oxide. Preferred heteroaryl groups include pyridyl, pyrimidyl, quinolinyl, indolyl, pyrrolyl, furanyl, thienyl, imidazolyl, pyrazolyl, indazolyl, thiazolyl and benzothiazolyl. The heteroaryl groups herein are unsubstituted or, when specified as "optionally substituted", can unless stated otherwise be substituted in one or more substitutable positions with various groups, as indicated.

The term "heterocycloalkyl" refers to a non-aromatic ring or ring system containing at least one heteroatom that is preferably selected from nitrogen, oxygen and sulfur, wherein said heteroatom is in a non-aromatic ring. The heterocycloalkyl may have 1, 2, 3 or 4 heteroatoms. The heterocycloalkyl may be saturated (i.e., a heterocycloalkyl) or partially unsaturated (i.e., a heterocycloalkenyl). Heterocycloalkyl includes monocyclic groups of three to eight annular atoms as well as bicyclic and polycyclic ring systems, including bridged and fused systems, wherein each ring includes three to eight annular atoms. The heterocycloalkyl ring is optionally fused to other heterocycloalkyl rings and/or non-aromatic hydrocarbon rings. In certain embodiments, the heterocycloalkyl groups have from 3 to 7 members in a single ring. In other embodiments, heterocycloalkyl groups have 5 or 6 members in a single ring. In some embodiments, the heterocycloalkyl groups have 3, 4, 5, 6 or 7 members in a single ring. Examples of heterocycloalkyl groups include, for example, azabicyclo[2.2.2]octyl (in each case also "quinuclidinyl" or a quinuclidine derivative), azabicyclo[3.2.1]octyl, 2,5-diazabicyclo[2.2.1]heptyl, morpholinyl, thiomorpholinyl, thiomorpholinyl S-oxide, thiomorpholinyl S,S-dioxide, 2-oxazolidonyl, piperazinyl, homopiperazinyl, piperazinonyl, pyrrolidinyl, azepanyl, azetidinyl, pyrrolinyl, tetrahydropyranyl, piperidinyl, tetrahydrofuranyl, tetrahydrothienyl, 3,4-dihydroisoquinolin-2(1*H*)-yl, isoindolindionyl, homopiperidinyl, homomorpholinyl, homothiomorpholinyl, homothiomorpholinyl S,S-dioxide, oxazolidinonyl, dihydropyrazolyl, dihydropyrrolyl, dihydropyrazinyl, dihydropyridinyl, dihydropyrimidinyl, dihydrofuryl, dihydropyranyl, imidazolidonyl, tetrahydrothienyl S-oxide, tetrahydrothienyl S,S-dioxide and homothiomorpholinyl S-oxide. Especially desirable heterocycloalkyl groups include morpholinyl, 3,4-dihydroisoquinolin-2(1*H*)-yl, tetrahydropyranyl, piperidinyl, aza-bicyclo[2.2.2]octyl, γ-butyrolactonyl (i.e., an oxo-substituted tetrahydrofuranyl), y-butryolactamyl (i.e., an oxo-substituted pyrrolidine), pyrrolidinyl, piperazinyl, azepanyl, azetidinyl, thiomorpholinyl, thiomorpholinyl S,S-dioxide, 2-oxazolidonyl, imidazolidonyl, isoindolindionyl, piperazinonyl. The heterocycloalkyl groups herein are unsubstituted or, when specified as "optionally substituted", can unless stated otherwise be substituted in one or more substitutable positions with various groups, as indicated.

The term "cycloalkyl" or "cycloalkane" refers to a non-aromatic carbocyclic ring or ring system, which may be saturated (i.e., a cycloalkyl, a cycloalkane) or partially unsaturated (i.e., a cycloalkenyl). The cycloalkyl ring can be optionally fused to or otherwise attached (e.g., bridged systems) to other cycloalkyl rings. Certain examples of cycloalkyl groups or cycloalkanes present in the disclosed compounds have from 3 to 7 members in a single ring, such as having 5 or 6 members in a single ring. In some embodiments, the cycloalkyl groups have 3, 4, 5, 6 or 7 members in a single ring. Examples of cycloalkyl groups include, for example, cyclohexyl, cyclopentyl, cyclobutyl, cyclopropyl, tetrahydronaphthyl and bicyclo[2.2.1]heptane. Examples of cycloalkanes include, for example, cyclohexane, methylcyclohexane, cyclohexanone, cyclohexanol, cyclopentane, cycloheptane, and cycloctane. The cycloalkyl groups herein are unsubstituted or, when specified as "optionally substituted", may be substituted in one or more substitutable positions with various groups, as indicated.

The term "ring system" encompasses monocycles, as well as fused and/or bridged polycycles.

The terms "halogen" or "halo" indicate fluorine, chlorine, bromine, and iodine. In certain embodiments of each and every embodiment described herein, the term "halogen" or "halo" refers to fluorine or chlorine. In certain embodiments of each and every embodiment described herein, the term "halogen" or "halo" refers to fluorine.

The term "halide" indicates fluoride, chloride, bromide, and iodide. In certain embodiments of each and every embodiment described herein, the term "halide" refers to bromide or chloride.

The term "substituted," when used to modify a specified group or radical, means that one or more hydrogen atoms of the specified group or radical are each, independently of one another, replaced with the same or different substituent groups as defined below, unless specified otherwise.

Specific protecting groups may be used to protect reactive functionalities of a starting material or intermediate to prepare a desired product. In general, the need for such protecting groups as well as the conditions necessary to attach and remove such groups will be apparent to those skilled in the art of organic synthesis. An authoritative account describing the many alternatives to the trained practitioner are J. F. W. McOmie, "Protective Groups in Organic Chemistry", Plenum Press, London and New York 1973, in T. W. Greene and P. G. M. Wuts, "Protective Groups in Organic Synthesis", Third edition, Wiley, New York 1999, in "The Peptides"; Volume 3 (editors: E. Gross and J. Meienhofer), Academic Press, London and New York 1981, in "Methoden der organischen Chemie", Houben-Weyl, 4.sup.th edition, Vol. 15/I, Georg Thieme Verlag, Stuttgart 1974, in H.-D. Jakubke and H. Jescheit, "Aminosauren, Peptide, Proteine", Verlag Chemie, Weinheim, Deerfield Beach, and Basel 1982, and/or in Jochen Lehmann, "Chemie der Kohlenhydrate: Monosaccharide and Derivate", Georg Thieme Verlag, Stuttgart 1974. The protecting groups may be removed at a convenient subsequent stage using methods known from the art.

As used herein, the term "benzyl" ("Bn") includes unsubstituted (i.e., (C₆H₅)-CH₂-) and substituted benzyl (i.e., benzyl substitututed at the 2-, 3-, and/or 4- position with C₁-C₈ alkyl or halide). The person of ordinary skill in the art will appreciate that oxygen protecting groups include alkoxycarbonyl, acyl, acetal, ether, ester, silyl ether, alkylsulfonyl, and arylsulfonyl. Exemplary oxygen protecting groups include allyl, triphenylmethyl (trityl or Tr), benzyl, methanesulfonyl, p-toluenesulfonyl, p-methoxybenzyl (PMB), p-methoxyphenyl (PMP), methoxymethyl (MOM), p-methoxyethoxymethyl (MEM), tetrahydropyranyl (THP), ethoxyethyl (EE),methylthiomethyl (MTM), 2-methoxy-2-propyl (MOP), 2-trimethylsilylethoxymethyl (SEM), benzoate (BZ), allyl carbonate, 2.2.2-trichloroethyl carbonate (Troc), 2-trimethylsilylethyl carbonate, trimethylsilyl (TMS), triethylsilyl (TES), triisopropylsilyl (TIPS), triphenylsilyl (TPS), t-butyldimethylsilyl (TBDMS), and t-butyldiphenylsilyl (TBDPS). A variety of protecting groups for the oxygen and the synthesis thereof may be found in "Protective Groups in Organic Synthesis" by T. W. Greene and P. G. M. Wuts, John Wiley & Sons, 1999. In certain embodiments, an appropriate oxygen protecting goup may be used in place of benzyl.

It is to be understood that the embodiments of the invention disclosed herein are illustrative of the principles of the present invention. Other modifications that may be employed are within the scope of the invention. Thus, by way of example, but not of limitation, alternative configurations of the present invention may be utilized in accordance with the teachings herein. Accordingly, the present invention is not limited to that precisely as shown and described.

### Chemical Synthesis of Buprenorphine

The disclosure relates to methods for preparing buprenorphine:

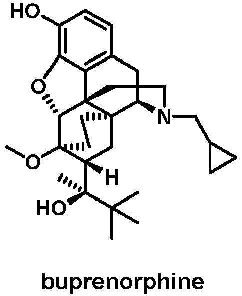

In various aspects and embodiments, the methods comprise a series of reaction steps to prepare buprenorphine from a compound of Formula I:

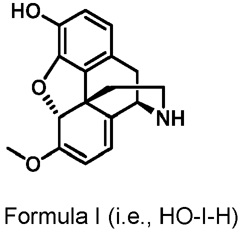

Such methods provide an improved route to buprenorphine that can be shorter, more efficient, and/or produce less toxic waste than, e.g., current commercial routes to buprenorphine. As a result, these aspects and embodiments can be well-suited for commercial (e.g., kg-scale) production of buprenorphine. Further, such methods advantageously avoid the harsh conditions and/or toxic byproducts of an N-demethylation step and can accordingly be particularly well-suited for producing buprenorphine on a commercial, e.g., kg, scale.

In various aspects and embodiments, the methods comprise a series of reaction steps to prepare buprenorphine from a compound of Formula I-H: 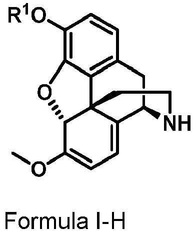 wherein R¹ is H (Compound HO-I-H; nororipavine).

In some embodiments, the methods comprise in a first solvent system S1 comprising a polar protic solvent reacting a compound of Formula I-H with benzyl halide, benzyl sulfonate, or activated benzyl alcohol to provide a compound of Formula I-Bn: 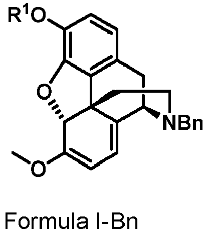 wherein R¹ is benzyl (Compound BnO-I-Bn). A preparation of Compound BnO-I-Bn, as an intermediate towards noroxymorphone and ultimately towards naltrexone and naloxone, was described in Helv. Chim. Acta 92:1359-65 (2009).

In some embodiments, the methods comprise in a second solvent system S2 comprising a polar protic solvent reacting a compound of Formula I-Bn with methyl vinyl ketone to provide a compound of Formula II-Bn: 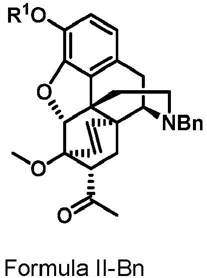 wherein R¹ is benzyl (Compound BnO-II-Bn).

In some embodiments, the methods comprise in a third solvent system S3 comprising a nonpolar solvent reacting a compound of Formula II-Bn with tert-butylmagnesium halide to provide a compound of Formula IIIA-Bn: 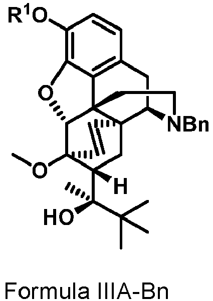 wherein R¹ is benzyl (Compound BnO-IIIA-Bn).

In some embodiments, the methods comprise reacting a compound of Formula IIIA-Bn with H₂ in the presence of a hydrogenation catalyst to provide a compound of Formula IV-H: 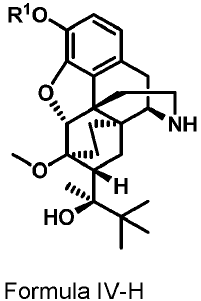 wherein R¹ is H (Compound HO-IV-H; norbuprenorphine).

In some embodiments, the methods comprise reacting a compound of Formula IV-H (e.g., Compound HO-IV-H) with cyclopropane carboxaldehyde followed by a hydride source; or reacting a compound of Formula IV-H (e.g., Compound HO-IV-H) with cyclopropanecarboxylic acid halide followed by a reducing agent; or reacting a compound of Formula IV-H (e.g., Compound HO-IV-H) with cyclopropylmethyl halide or activated cyclopropane methanol; to provide buprenorphine.

### Formula I-H → Formula I-Bn

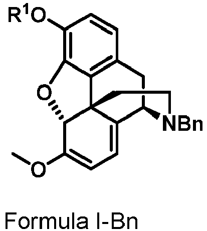

| R¹ of Formula I-Bn | Compound |
|---|---|
| Bn | Compound BnO-I-Bn |

### Step (i)(F)

In some embodiments, reacting a compound of Formula I-H with benzyl halide, benzyl sulfonate, or activated benzyl alcohol (*e.g.,* activated with a sulfonate group such as a *p*-toluene sulfonyl group or a methyl sulfonyl group, or with triphenylphosphine) in a first solvent system S1 comprising a polar protic solvent provides a compound of Formula I-Bn. In certain embodiments, reacting Compound HO-I-H with benzyl halide, benzyl sulfonate, or activated benzyl alcohol provides Compound BnO-I-Bn. See Example 1.

In some embodiments, the benzyl halide is benzyl chloride or benzyl bromide. In some embodiments, the reaction is performed in the presence of a strong base, *e.g.,* an alkali metal hydride. In some embodiments, the first solvent system S1 has a dielectric constant of at least about 12, or at least about 13, or at least about 14. In various other embodiments as described herein, the dielectric constant of S1 is at least about 15, or at least about 16, or at least about 18, or at least about 20.

In certain embodiments as otherwise described herein, S1 comprises at least about 50 vol.% of at least one protic solvent having a dielectric constant of at least about 12. In various other embodiments, the at least one protic solvent is present in an amount of at least 60 vol.%, or at least 70 vol.%, or at least 75 vol.%, or at least 80 vol.%, or at least 90 vol.%, or at least 95 vol.%. In certain embodiments, the at least one protic solvent has a dielectric constant of at least 13, or at least 14, or at least 15, or at least 16, or at least 18, or at least 20.

In some embodiments, S1 comprises at least one protic solvent having a polarity index of at least about 3, or at least about 3.5, or at least about 3.75, or at least about 4. As used herein, the solvent polarity index of a solvent can be determined according to Snyder, e.g., as reported in Snyder, L.R. "Classification of the Solvent Properties of Common Liquids." J. Chromatogr. (1978) 16:6, 223-234. In various embodiments, S1 comprises at least about 50 vol.%, or at least about 75 vol.%, or at least about 90 vol.% of at least one protic solvent having a polarity index of at least about 3, or at least 3.5, or at least 3.75, or at least 4.

In some embodiments as otherwise described herein, S1 comprises a C₁-C₄ alcohol (e.g., methanol, ethanol, n-propanol, isopropanol, n-butanol, or sec-butanol) and optionally water. In various embodiments, S1 comprises about 50-100 vol.% isopropanol and 0-50 vol.% water.

In some embodiments, the benzyl halide, benzyl sulfonate, or activated benzyl alcohol is reacted at a temperature within the range of about -20°C to about 40°C, *e.g.,* about -20°C to about 35°C, or about -20°C to about 30°C, or about -20°C to about 25°C, or about - 20°C to about 20°C, or about -20°C to about 15°C, or about -20°C to about 10°C, or about - 20°C to about 5°C, or about -20°C to about 0°C, or about -15°C to about 40°C, or about -10°C to about 40°C, or about -5°C to about 40°C, or about 0°C to about 40°C, or about 5°C to about 20°C, or about 10°C to about 40°C, or about 15°C to about 40°C, or about 20°C to about 40°C, or about -15°C to about 35°C, or about -10°C to about 30°C, or about -5°C to about 25°C, or about 0°C to about 20°C, or about 5°C to about 15°C. In some embodiments, the benzyl halide, benzyl sulfonate, or activated benzyl alcohol is reacted for a period of time within the range of about 1h to about 2 days, *e.g.,* 2h to about 2 days, 3h to about 2 days, 6 hours to about 2 days, about 12 hours to about 2 days, or about 18 hours to about 2 days, or about 1 day to about 2 days, or about 1.25 days to about 2 days, or about 1.5 days to about 2 days, or about 6 hours to about 1.75 days, or about 6 hours to about 1.5 days, or about 6 hours to about 1.25 days, or about 6 hours to about 1 day, or about 6 hours to about 18 hours, or about 12 hours to about 1.75 days, or about 18 hours to about 1.5 days, or about 1h to about 1 day, or about 1h to about 12h, or about 1h to about 6h, or about 1h to about 4h.

### Formula I-Bn → Formula II-Bn

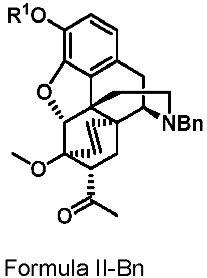

| R¹ of Formula II-Bn | Compound |
|---|---|
| Bn | Compound BnO-II-Bn |

### Step (ii)(B)

In some embodiments, reacting a compound of Formula I-Bn with methyl vinyl ketone in a second solvent system S2 comprising a polar protic solvent provides a compound of Formula II-Bn. In certain embodiments, reacting Compound BnO-I-Bn with methyl vinyl ketone provides Compound BnO-II-Bn. *See* Example 3.

In some embodiments, the methyl vinyl ketone is reacted at a temperature within the range of about 40°C to about 120°C, e.g., about 45°C to about 120°C, or about 50°C to about 120°C, or about 55°C to about 120°C, or about 60°C to about 120°C, or about 65°C to about 120°C, or about 70°C to about 120°C, or about 75°C to about 120°C, or about 80°C to about 120°C, or about 85°C to 120°C, or about 90°C to about 120°C, or about 40°C to about 115°C, or about 40°C to about 110°C, or about 40°C to about 105°C, or about 40°C to about 100°C, or about 40°C to about 95°C, or about 40°C to about 90°C, or about 40°C to about 85°C, or about 40°C to about 80°C, or about 40°C to about 75°C, or about 40°C to about 70°C, or about 45°C to about 115°C, or about 50°C to about 110°C, or about 55°C to about 105°C, or about 60°C to about 100°C, or about 65°C to about 95°C, or about 70°C to about 90°C. In some embodiments, the methyl vinyl ketone is reacted for a period of time within the range of about 2 hours to about 2 days, *e.g.,* about 4 hours to about 2 days, or about 6 hours to about 2 days, or about 12 hours to about 2 days, or about 18 hours to about 2 days, or about 1 days to about 2 days, or about 1.25 days to about 2 days, or about 1.5 days to about 2 days, or about 2 hours to about 1.75 days, or about 2 hours to about 1.5 days, or about 2 hours to about 1.25 days, or about 2 hours to about 1 day, or about 2 hours to about 18 hours, or about 2 hours to about 12 hours, or about 4 hours to about 1.75 days, or about 6 hours to about 1.5 days, or about 12 hours to about 1.25 days, or about 18 hours to about 1 day.

In certain embodiments as otherwise described herein, the reaction of (ii)(B) is carried out under oxygen, e.g., a mixture of inert gas and oxygen having a different composition than that of air. In certain embodiments, the reaction is carried out in an atmosphere wherein inert gas (e.g., argon) is present as greater than 5 vol.% (e.g., greater than 20 vol.%, or greater than 50 vol. %), and and oxygen is present as less than 25 vol.% (e.g., less than 21 vol.%, or less than 20 vol.%, or less than 10 vol.%, or less than 5 vol.%). In certain embodiments, the reaction is carried out in an atmosphere wherein oxygen is present at between 1 vol.% and about 21 vol.%, or between 3 vol.% and 20 vol.%, or between 5 vol. % and 20 vol.%, or between 10 vol.% and 20 vol.%, or between 1 vol.% and 20 vol.%, or between 1 vol.% and 15 vol.%, or between 1 vol.% and 10 vol.%, or between 1 vol.% and 7 vol.%, or between 1 vol.% and 5 vol.%. In certain other embodiments, the reaction is performed in substantially inert atmosphere (e.g., oxygen is present at less than 0.1 vol. %, or less than 0.01 vol. %, or less than 0.001 vol. %).

In certain other embodiments, the reaction of (ii)(B) is carried out under a mixture of gases approximately the same as air (e.g., dry air). In other embodiments, the reaction is carried out wherein the ratio of inert gas to gaseous oxygen is approximately 79 vol.% to 21 vol.%.

It has been found that the use of some amount of oxygen in the atmosphere of the reaction (ii)(B) serves to increase the yield of the reaction. Without wishing to be bound by theory, it is presently believed that trace oxygen prevents methyl vinyl ketone polymerization, allowing for additional methyl vinyl ketone monomers to be present as reactants. This reduces the equivalents of methyl vinyl ketone necessary to propel the reaction to completion, and can also increase the reaction yield. Beyond reducing the need for excess methyl vinyl ketone, providing a reaction atmosphere containing at least some oxygen generally requires less rigorous reaction condition and equipment, especially at scale, as rigorous oxygen exclusion is no longer required. Together, this development allows for a more efficient synthetic protocol and enhanced reaction yields with lower precursor and capital expenditures.

In some embodiments, the second solvent system S2 has a dielectric constant of at least about 12, or at least about 13, or at least about 14. In various other embodiments as described herein, the dielectric constant of S2 is at least about 15, or at least about 16, or at least about 18, or at least about 20.

In certain embodiments as otherwise described herein, S2 comprises at least about 50 vol.% of at least one protic solvent having a dielectric constant of at least about 12. In various other embodiments, the at least one protic solvent is present in an amount of at least 60 vol.%, or at least 70 vol.%, or at least 75 vol.%, or at least 80 vol.%, or at least 90 vol.%, or at least 95 vol.%. In certain embodiments, the at least one protic solvent has a dielectric constant of at least 13, or at least 14, or at least 15, or at least 16, or at least 18, or at least 20.

In some embodiments, S2 comprises at least one protic solvent having a polarity index of at least about 3, or at least about 3.5, or at least about 3.75, or at least about 4. In various embodiments, S2 comprises at least about 50 vol.%, or at least about 75 vol.%, or at least about 90 vol.% of at least one protic solvent having a polarity index of at least about 3, or at least 3.5, or at least 3.75, or at least 4.

In some embodiments as otherwise described herein, S2 comprises a C₁-C₄ alcohol (e.g., methanol, ethanol, n-propanol, isopropanol, n-butanol, or sec-butanol) and optionally water. In various embodiments, S2 comprises about 50-100 vol.% isopropanol and 0-50 vol.% water. In certain desirable embodiments, S2 has substantially the same composition as S1, described above.

### Telescoped synthesis: Formula I-H → Formula II-Bn

In certain desirable embodiments, reactions (i)(F) and (ii)(B) can be performed sequentially, advantageously without intervening purification and without substantial removal of solvent system S1. In certain embodiments as otherwise described herein, S2 has substantially the same composition as S1. In such embodiments, the reactants and purification steps of step (ii)(B) comprise the crude reaction product of step (i)(F). Accordingly, in certain embodiments as otherwise described herein, the methyl vinyl ketone of step (ii)(B) is added to a crude reaction product of step (i)(F), the crude reaction product comprising solvent S1 and Compound BnO-II-Bn. See Example 4.

As noted above, the reaction steps of (ii)(B) can be carried out without substantial removal of solvent or purification (e.g. chromatography). However, the person of ordinary skill in the art will appreciate that it may be necessary to adjust the pH of the crude reaction product of step (i)(F) before performing step (ii)(B). The pH may be may be adjusted with a wide variety of acids known in the art. For example, in certain embodiments, the pH is adjusted with the addition of acetic acid or hydrochloric acid. For example, the pH may be adjusted with a water-diluted acid such as 10% acetic acid, or 10% hydrochloric acid. In various embodiments as otherwise described herein, the pH is adjusted to be about neutral, e.g., between 6 and 8.

### Formula II-Bn → Formula IIIA-Bn

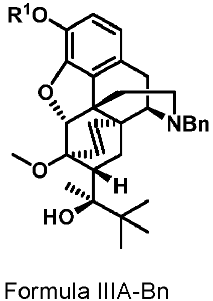

| R¹ of Formula IIIA-Bn | Compound |
|---|---|
| Bn | Compound BnO-IIIA-Bn |

### Step (iii)(D)

In some embodiments, reacting a compound of Formula II-Bn with a *tert-*butylmagnesium compound in a third solvent system S3 comprising a nonpolar solvent provides a compound of Formula IIIA-Bn. In certain embodiments, reacting Compound BnO-II-Bn with a *tert*-butylmagnesium compound provides Compound BnO-IIIA-Bn. See Example 5.

In some embodiments, the *tert*-butylmagnesium compound is a *tert-*butylmagnesium halide. For example, the *tert*-butylmagnesium compound is *tert-*butylmagnesium chloride or *tert*-butylmagnesium bromide. In some embodiments, the reaction is performed in a solvent comprising a nonpolar solvent, *e.g., tert*-butylmethyl ether, 2-methyltetrahydrofuran, diethyl ether, dimethoxymethane, benzene, toluene, or a mixture of thereof.

In some embodiments, the *tert*-butylmagnesium compound is reacted at a temperature within the range of about 15°C to about 100°C, *e.g.,* about 20°C to about 100°C, or about 25°C to about 100°C, or about 30°C to about 100°C, or about 15°C to about 95°C, or about 15°C to about 90°C, or about 15°C to about 85°C, or about 20°C to about 95°C, or about 25°C to about 90°C. In some embodiments, the *tert*-butylmagnesium halide is reacted for a period of time within the range of about 30 minutes to about 8 hours, e.g., about 1 hours to about 8 hours, or about 1.5 hours to about 8 hours, or about 2 hours to about 8 hours, or about 2.5 hours to about 8 hours, or about 3 hours to about 8 hours, or about 3.5 hours to about 8 hours, or about 4 hours to about 8 hours, or about 4.5 hours to about 8 hours, or about 5 hours to about 8 hours, or about 30 minutes to about 7.5 hours, or about 30 minutes to about 7 hours, or about 30 minutes to about 6.5 hours, or about 30 minutes to about 6 hours, or about 30 minutes to about 5.5 hours, or about 30 minutes to about 5 hours, or about 30 minutes to about 4.5 hours, or about 30 minutes to about 4 hours, or about 30 minutes to about 3.5 hours, or about 1 hour to about 7.5 hours, or about 1.5 hours to about 7 hours, or about 2 hours to about 6.5 hours, or about 2.5 hours to about 6 hours, or about 3 hours to about 5.5 hours.

As described above, the third solvent system S3 comprises a nonpolar solvent. In certain embodiments as otherwise described herein, the third solvent system S3 comprises at least one at least one nonpolar solvent having a dielectric constant of at most about 8, or at most about 7, or at most about 6, or at most about 5, or at most about 4, or at most about 3. For example, in various embodiments, S3 comprises at least 60 vol.%, or at least 70 vol.%, or at least 75 vol.%, or at least 80 vol.%, or at least 90 vol.%, or at least 95 vol.% of the at least one nonpolar solvent having a dielectric constant of at most 8, or at most 7, or at most 6, or at most 5, or at most 4, or at most 3. In further embodiments, the nonpolar solvent that comprises S3 has a polarity index of less than 4, or less than 3, or less than 2, or less than 1. For example, in various embodiments, S3 comprises at least 60 vol.%, or at least 70 vol.%, or at least 75 vol.%, or at least 80 vol.%, or at least 90 vol.%, or at least 95 vol.% of the at least one nonpolar solvent has a polarity index of less than 4, or less than 3, or less than 2, or less than 1.

In certain desirable embodiments, polar solvents or solvents with large dielectric constants are not substantially present in S3, or are present in S3 in a relatively small amount. For example, in certain embodiments, S3 comprises less than about 20 vol.%, or less than about 10 vol.%, or less than about 5 vol.%, or less than about 1 vol.% of a total amount of solvents having a dielectric constant of greater than 4, or greater than 6, or greater than 8. In various embodiments as otherwise described herein, S3 comprises less than about 20 vol.%, or less than about 10 vol.%, or less than about 5 vol.%, or less than about 1 vol.% of a total amount of solvents having a polarity index of 2 or greater, or 3 or greater, or 4 or greater.

In some embodiments, S3 comprises 30-90 vol.% of one or more C₅-C₁₀ alkanes and/or C₅-C₁₀ cycloalkanes. In certain embodiments, the alkanes and/or cycloalkanes are substituted (e.g., perfluorocyclohexane, perfluorohexane, etc.). For example, in certain embodiments the one or more alkanes and/or cycloalkanes include cyclohexane. In other embodiments, the one or more alkanes and/or cycloalkanes is cyclohexane. For example, S3 may comprise 10-50 vol.% toluene (e.g., 20-50 vol.% toluene, or 30-50 vol.% toluene), 30-90 vol.% cyclohexane (e.g., 40-90 vol.% cyclohexane, or 40-70 vol.% cyclohexane), and up to 30 vol% tetrahydrofuran (e.g., up to 20 vol.% tetrahydrofuran, or up to 10 vol.% tetrahydrofuran, or up to 5 vol.% tetrahydrofuran).

It is known in the art that certain Grignard reagents (e.g., *tert*-butylmagnesium halide) disproportionate to the *bis*-alkyl and *bis*-halide species, with the disproportionation favored under certain reaction and solvent conditions (e.g. a more polar solvent). Without wishing to be bound by theory, the present inventors believe that the reaction conditions of step (iii)(D) described herein can increase the concentration of the *bis* adducts present in solution, advantageously improving the yield of Formula IIIA-Bn (e.g., Compound BnO-IIIA-Bn). Accordingly, in certain embodiments, the *tert*-butylmagnesium compound comprises one or both of a *tert*-butylmagnesium halide and di-*tert*-butylmagnesium. For example, in some embodiments, the *tert*-butylmagnesium compound comprises a *tert*-butylmagnesium halide and di-*tert*-butylmagnesium. In certain embodiments, a proportion of the magnesium dihalide (e.g., magnesium dichloride) precipitates from solution. For example, substantially all of the magnesium dihalide may precipitate from solution. Alternatively, in certain other embodiments, there is essentially no precipitate formed from the Grignard reagent.

### Formula IIIA-Bn → Formula IV-H

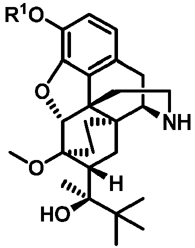

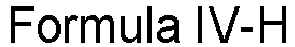

| R¹ of Formula IV-H | Compound |
|---|---|
| H | HO-IV-H |

### Step (iv)(C)

In some embodiments, reacting a compound of Formula IIIA-Bn with H₂ in the presence of a hydrogenation catalyst provides a compound of Formula IV-Bn. In certain embodiments, reacting Compound BnO-IIIA-Bn with H₂ in the presence of a hydrogenation catalyst provides Compound HO-IV-H. See Example 6.

In some embodiments, the hydrogenation catalyst comprises nickel, palladium, platinum, rhodium, or ruthenium. In some embodiments, the hydrogenation catalyst comprises platinum or palladium, supported on carbon. In some embodiments, the reaction is performed in a solvent comprising a polar protic or aprotic solvent, *e.g., n*-butanol, isopropanol, ethanol, methanol, *N*-methylpyrrolidone, tetrahydrofuran, ethyl acetate, acetone, dimethylformamide, acetonitrile, dimethylsulfoxide, propylene carbonate, or a mixture thereof.

In some embodiments, the hydrogen is reacted at a temperature within the range of about 15°C to about 120°C, e.g., about 20°C to about 120°C, or about 30°C to about 120°C, or about 40°C to about 120°C, or about 15°C to about 115°C, or about 20°C to about 110°C, or about 30°C to about 105°C, or about 40°C to about 115°C, or about 50°C to about 110°C. In some embodiments, the hydrogen is reacted for a period of time within the range of about 6 hours to about 3 days, *e.g.,* about 12 hours to about 3 days, or about 18 hours to about 3 days, or about 1 day to about 3 days, or about 1.25 days to about 3 days, or about 1.5 days to about 3 days, or about 6 hours to about 2.75 days, or about 6 hours to about 2.5 days, or about 6 hours to about 2.25 days, or about 6 hours to about 2 day, or about 6 hours to about 36 hours, or about 12 hours to about 2.5 days, or about 24 hours to about 2 days. In some embodiments, the hydrogen is reacted at a pressure within the range of about 1 atm to about 3 atm, e.g., about 1.25 atm to about 3 atm, or about 1.5 atm to about 3 atm, or about 1.75 atm to about 3 atm, or about 2 atm to about 3 atm, or about 1 atm to about 2.75 atm, or about 1 atm to about 2.5 atm, or about 1 atm to about 2.25 atm, or about 1 atm to about 2 atm, or about 1.25 atm to about 2.75 atm, or about 1.5 atm to about 2.5 atm, or about 1.75 atm to about 2.25 atm.

### Formula IV-H → Formula IV-MCP

### Step (v)(A1)

In some embodiments, reacting a compound of Formula IV-H with cyclopropane carboxaldehyde followed by a hydride source provides a compound of Formula IV-MCP. In certain embodiments, reacting Compound HO-IV-H with cyclopropane carboxaldehyde followed by a hydride source provides buprenorphine. *See* Example 7.

In some embodiments, the hydride source is formic acid, hydrogen, sodium cyanoborohydride, sodium borohydride, or sodium triacetoxy borohydride. In some embodiments, the hydride source is formic acid. In some embodiments, the reaction is catalyzed by a ruthenium(I) complex or a ruthenium(II) complex, *e.g.,* a dichloro(p-cymene)ruthenium(II) dimer. In some embodiments, the reaction is performed in a solvent comprising a polar aprotic solvent, e.g., *N*-methylpyrrolidone, tetrahydrofuran, ethyl acetate, acetone, dimethylformamide, acetonitrile, dimethylsulfoxide, propylene carbonate, or a mixture thereof. In some embodiments, the reaction is performed in the presence of a trialkylamine, *e.g.,* triethylamine, diisopropylethylamine, 4-methyl-morpholine, or *N*-methyl-piperidine.

In some embodiments, the cyclopropane carboxaldehyde is reacted at a temperature within the range of about 30°C to about 90°C, e.g., about 35°C to about 90°C, or about 40°C to about 90°C, or about 45°C to about 90°C, or about 50°C to about 90°C, or about 55°C to about 90°C, or about 60°C to about 90°C, or about 65°C to about 90°C, or about 70°C to about 90°C, or about 30°C to about 85°C, or about 30°C to about 80°C, or about 30°C to about 75°C, or about 30°C to about 70°C, or about 30°C to about 65°C, or about 30°C to about 60°C, or about 30°C to about 55°C, or about 30°C to about 50°C, or about 35°C to about 85°C, or about 40°C to about 80°C, or about 45°C to about 75°C, or about 50°C to about 70°C, or about 55°C to about 65°C. In some embodiments, the cyclopropane carboxaldehyde is reacted for a period of time within the range of about 30 minutes to about 5 hours, e.g.,about 1 hour to about 5 hours, or about 1.5 hours to about 5 hours, or about 2 hours to about 5 hours, or about 2.5 hours to about 5 hours, or about 3 hours to about 5 hours, or about 3.5 hours to about 5 hours, or about 4 hours to about 5 hours, or about 30 minutes to about 4.5 hours, or about 30 minutes to about 4 hours, or about 30 minutes to about 3.5 hours, or about 30 minutes to about 3 hours, or about 30 minutes to about 2.5 hours, or about 30 minutes to about 2 hours, or about 30 minutes to about 1.5 hours.

### Step (v)(A2)

In some embodiments, reacting a compound of Formula IV-H with cyclopropanecarboxylic acid halide followed by a reducing agent provides a compound of Formula IV-MCP. In certain embodiments, reacting Compound HO-IV-H with cyclopropanecarboxylic acid halide followed by a reducing agent provides buprenorphine.

In some embodiments, the cyclopropanecarboxylic acid halide is cyclopropanecarboxylic acid chloride, cyclopropanecarboxylic acid anhydride, cyclopropanecarboxylic acid bromide, or an activated cyclopropanecarboxylic acid (*e.g.,* an activated cyclopropanecarboxylic acid formed by reaction with an alcohol such as pentafluorophenol, 4-nitrophenol, *N*-hydroxysuccinimide, *N*-hydroxymaleimide, 1-Hydroxybenzotriazole, or 1-hydroxy-7-azabenzotriazole). In some embodiments, the reducing agent is LiAlH₄ or NaBH₄. In some embodiments, the reaction with cyclopropanecarboxylic acid halide is performed in a solvent comprising a nonpolar solvent, *e.g.,* dichloromethane, chloroform, toluene, 1,4-dioxane, diethyl ether, benzene, or a mixture thereof. In some embodiments, the reaction with a reducing agent is performed in a solvent comprising a polar aprotic solvent, *e.g., N*-methylpyrrolidone, tetrahydrofuran, ethyl acetate, acetone, dimethylformamide, acetonitrile, dimethylsulfoxide, propylene carbonate, or a mixture thereof.

In some embodiments, the cyclopropanecarboxylic acid halide is reacted at a temperature within the range of about -20°C to about 40°C, *e.g.,* about -20°C to about 35°C, or about -20°C to about 30°C, or about -20°C to about 25°C, or about -20°C to about 20°C, or about -20°C to about 15°C, or about -20°C to about 10°C, or about -20°C to about 5°C, or about -20°C to about 0°C, or about -15°C to about 40°C, or about -10°C to about 40°C, or about -5°C to about 40°C, or about 0°C to about 40°C, or about 5°C to about 20°C, or about 10°C to about 40°C, or about 15°C to about 40°C, or about 20°C to about 40°C, or about - 15°C to about 35°C, or about -10°C to about 30°C, or about -5°C to about 25°C, or about 0°C to about 20°C, or about 5°C to about 15°C. In some embodiments, the cyclopropanecarboxylic acid halide is reacted for a period of time within the range of about 6 hours to about 2 days, *e.g.,* about 12 hours to about 2 days, or about 18 hours to about 2 days, or about 1 day to about 2 days, or about 1.25 days to about 2 days, or about 1.5 days to about 2 days, or about 6 hours to about 1.75 days, or about 6 hours to about 1.5 days, or about 6 hours to about 1.25 days, or about 6 hours to about 1 day, or about 6 hours to about 18 hours, or about 12 hours to about 1.75 days, or about 18 hours to about 1.5 days. In some embodiments, the reducing agent is reacted at a temperature within the range of about 35°C to about 85°C, *e.g.,* about 40°C to about 85°C, or about 45°C to about 85°C, or about 50°C to about 85°C, or about 55°C to about 85°C, or about 60°C to about 85°C, or about 65°C to about 85°C, or about 35°C to about 80°C, or about 35°C to about 75°C, or about 35°C to about 70°C, or about 35°C to about 65°C, or about 35°C to about 60°C, or about 35°C to about 55°C, or about 40°C to about 80°C, or about 45°C to about 75°C, or about 50°C to about 70°C, or about 55°C to about 65°C. In some embodiments, the reducing agent is reacted for a period of time within the range of about 5 minutes to about 3 hours, *e.g.,* or about 10 minutes to about 3 hours, or about 15 minutes to about 3 hours, or about 30 minutes to about 3 hours, or about 45 minutes to about 3 hours, or about 1 hour to about 3 hours, or about 1.25 hours to about 3 hours, or about 1.5 hours to about 3 hours, or about 1.75 hours to about 3 hours, or about 2 hours to about 3 hours, or about 5 minutes to about 2.75 hours, or about 5 minutes to about 2.5 hours, or about 5 minutes to about 2.25 hours, or about 5 minutes to about 2 hours, or about 5 minutes to about 1.75 hours, or about 5 minutes to about 1.5 hours, or about 5 minutes to about 1.25 hours, or about 5 minutes to about 1 hour, or about 10 minutes to about 2.75 hours, or about 15 minutes to about 2.5 hours, or about 30 minutes to about 2.25 hours, or about 45 minutes to about 2 hours, or about 1 hour to about 1.75 hours.

### Step (v)(A3)

In some embodiments, reacting a compound of Formula IV-H with cyclopropylmethyl halide or activated cyclopropane methanol (*e.g.,* activated with a sulfonate group such as a *p*-toluene sulfonyl group or a methyl sulfonyl group, or with triphenylphosphine) provides a compound of Formula IV-MCP. In certain embodiments, reacting Compound HO-IV-H with cyclopropylmethyl halide or activated cyclopropane methanol provides buprenorphine.

In some embodiments, the cyclopropylmethyl halide is cyclopropylmethyl chloride or cyclopropylmethyl bromide. In some embodiments, the reaction is performed in the presence of a trialkylamine, *e.g.,* triethylamine, diisopropylethylamine, 4-methyl-morpholine, or *N*-methyl-piperidine. In some embodiments, the reaction is performed in a solvent comprising a polar protic solvent, *e.g., n*-butanol, isopropanol, ethanol, methanol, water, or a mixture thereof.

In some embodiments, the cyclopropylmethyl halide or activated cyclopropane methanol is reacted at a temperature within the range of about 40°C to about 120°C, *e.g.,* about 45°C to about 120°C, or about 50°C to about 120°C, or about 55°C to about 120°C, or about 60°C to about 120°C, or about 65°C to about 120°C, or about 70°C to about 120°C, or about 75°C to about 120°C, or about 80°C to about 120°C, or about 85°C to 120°C, or about 90°C to about 120°C, or about 40°C to about 115°C, or about 40°C to about 110°C, or about 40°C to about 105°C, or about 40°C to about 100°C, or about 40°C to about 95°C, or about 40°C to about 90°C, or about 40°C to about 85°C, or about 40°C to about 80°C, or about 40°C to about 75°C, or about 40°C to about 70°C, or about 45°C to about 115°C, or about 50°C to about 110°C, or about 55°C to about 105°C, or about 60°C to about 100°C, or about 65°C to about 95°C, or about 70°C to about 90°C. In some embodiments, the cyclopropylmethyl halide or activated cyclopropane methanol is reacted for a period of time within the range of about 30 minutes to about 6 hours, *e.g.,* about 1 hours to about 6 hours, or about 1.5 hours to about 6 hours, or about 2 hours to about 6 hours, or about 2.5 hours to about 6 hours, or about 3 hours to about 6 hours, or about 3.5 hours to about 6 hours, or about 4 hours to about 6 hours, or about 30 minutes to about 5.5 hours, or about 30 minutes to about 5 hours, or about 30 minutes to about 4.5 hours, or about 30 minutes to about 4 hours, or about 30 minutes to about 3.5 hours, or about 30 minutes to about 3 hours, or about 30 minutes to about 2.5 hours, or about 1 hours to about 5.5 hours, or about 1.5 hours to about 5 hours, or about 2 hours to about 4.5 hours, or about 2.5 hours to about 4 hours.

### Formula I-H → Buprenorphine

In another aspect, the method of preparing buprenorphine comprises the series of steps provided in Table 1:

**Table 1. 5-step buprenorphine route**

| No. | Substrate | Step | Product |
|---|---|---|---|
| i | Compound HO-I-H | (F) | Compound BnO-I-Bn |
| ii | Compound BnO-I-Bn | (B) | Compound BnO-II-Bn |
| iii | Compound BnO-II-Bn | (D) | Compound BnO-IIIA-Bn |
| iv | Compound BnO-IIIA-Bn | (C) | Compound HO-IV-H |
| v | Compound HO-IV-H | (A1), (A2), or (A3) | buprenorphine |

The person of ordinary skill in the art will appreciate that additional steps such as, for example, purification (*e.g.,* crystallization) or formation of an addition salt (*e.g.,* formation of buprenorphine-HCl) may be included in the methods of the disclosure as otherwise described herein.

The invention will be further described in the following examples, which do not limit the scope of the invention described in the claims.

### EXAMPLES

The Examples that follow are illustrative of specific embodiments of the invention, and various uses thereof. They are set forth for explanatory purposes only, and are not to be taken as limiting the invention.

Reagents and solvents used in the Examples provided below are reagent grade (or higher) commercial products. Water utilized in the Examples was de-ionized. Northebaine and nororipavine were prepared according to literature procedures.

### Preparation of buprenorphine from nororipavine

### Example 1. Preparation of Compound BnO-I-Bn (step F)

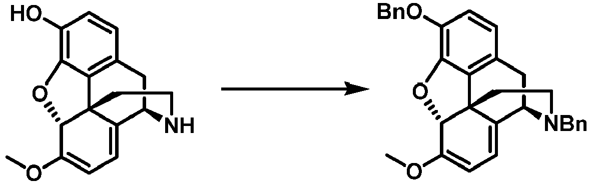

A 500 mL flask was charged with nororipavine (1.00 g, 3.5 mmol), iPrOH (20 mL), and water (10 mL). The suspension was stirred at room temperature and NaOH-pellets (0.42g, 10.6 mmol, 3 equiv) were added. After 10 min a light brown solution was obtained and benzyl bromide (1.50 g, 8.8 mmol, 2.5 equiv) was added over a period of 1 min. A slight exotherm was observed and after 10 min a precipitate was formed. After 2 h to the mixture was added water (20 mL). The resulting suspension was cooled in ice-water for 1 h and then filtered. The solid was washed with water (2x10 mL) and dried under vacuum to afford Compound BnO-I-Bn (1.6 g, 96%). Analytical data were in agreement with the literature.

### Example 2. Solvent screening optimization for Preparation of Compound BnO-II-Bn

It is known that Diels-Alder reactions often result in a mixture of two adducts. It was hypothesized that this may contribute to poor yields observed by the present inventors in the synthesis of Compound BnO-II-Bn. It was further hypothesized that solvent may be an effective variable to influence the relative proportions of diastereomerers produced and can thereby be modified to favor the desired product. To test this hypothesis, a solvent screening experiment was carried out with the typical solvent (Toluene) and several test solvents. Notably, the test solvents were designed to be polar solvents, in contrast to toluene. The results are summarized in Table 2:

**Table 2. Solvent Effects on Synthesis Yield**

| Exp. | Solvent | MVK equiv. | 2h s.m. **major** minor | 5h s.m. **major** minor | 20 h s.m. **major** minor | 26 h s. m. **major** minor | 74 h s.m. **major** minor |
|---|---|---|---|---|---|---|---|
| A | iPrOH | 16 | | | 0.0 | | |
| | | | 26.2 | 3.45 | **86.4** | | |
| | | | **59.6** | **82.0** | 2.24 (isomer ratio: 38) | | |
| | | | 1.34 | 2.00 | | | |
| B | 33%water | 16 | | | | 0.0 | |
| | | | 13.1 | 1.35 | 0.30 | **84.2** | |
| | 67% iPrOH | | **71.7** | **83.4** | **83.4** | 1.40 (isomer ratio: 60) | |
| | | | 1.02 | 1.36 | 1.45 | | |
| C | 33%water | 4 | | | | | 0.0 |
| | | | 86.2 | 50.8 | 4.00 | 1.65 | **83.8** |
| | 67% iPrOH | | **13.9** | **43.2** | **82.6** | **84.1** | 1.41 (isomer ratio: 59) |
| | | | 0.0 | 0.94 | 1.32 | 1.25 | |
| D | iPrOAc | 16 | | | | | 0.0 |
| | | | 69.2 | 50.0 | 1.87 | 1.37 | **82.5** |
| | | | **19.4** | **36.3** | **79.0** | **83.2** | 3.76 (isomer ratio 22) |
| | | | 0.81 | 1.65 | 3.76 | 3.65 | |
| ref. | Toluene | 20 | | | 0.0 | | |
| | | | | | **93.8** | | |
| | | | | | 4.4 (isomer ratio 21) | | |

| | | | | | | | |
|---|---|---|---|---|---|---|---|
| s.m.: starting material. | | | | | | | |

### Example 3. Preparation of Compound BnO-II-Bn (step B)

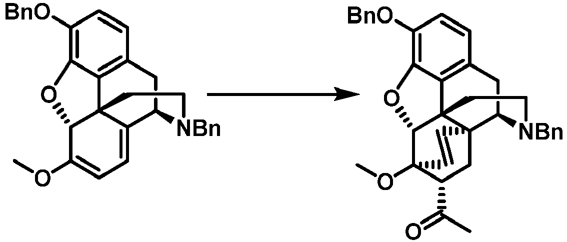

A solution of Compound BnO-I-Bn (1.54 g, 3.3 mmol) was suspended in 15 mL iPrOH and 5 mL of toluene and heated to 85 °C for 1 h. Next, methyl vinyl ketone (1.2 mL, 13.3 mmol) was added dropwise. After 20 h, the reaction mixture was cooled to room temperature and solvent removed under vacuum. The target material was purified by column chromatography (120 g SiO₂, elution with 0-20% EtOAc in heptane, R_{f} 0.3) to afford Compound BnO-II-Bn as a colorless solid (1.75 g, 93% yield).

### 7α-Acetyl-N,O-dibenzyl-6,14-endo(etheno)tetrahydro-nororipavine

Analytical data were in agreement with the literature.

### Example 4. Telescoped preparation of Compound BnO-II-Bn

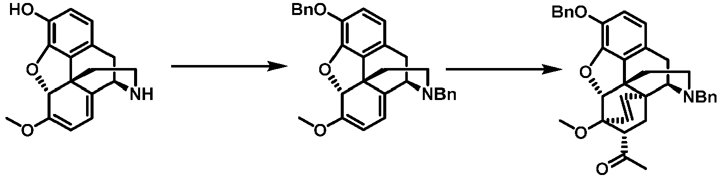

A 500 mL round bottom flask was charged with nororipavine (1.00 g, 3.5 mmol), iPrOH (15 mL), and water (6 mL). The suspension was stirred at room temperature and NaOH pellets (0.42 g, 10.6 mmol, 3 eq.) added. After 10 min a light brown solution was obtained and benzyl bromide (1.50 g, 8.8 mmol, 2.5 eq.) was added over a period of 1 min. A slight exotherm was observed and after 10 min a precipitate was formed. The mixture was stirred for 3 h and then the pH adjusted from basic to a pH of 7.3 by addition of a 10% acetic acid solution. The resulting suspension was heated to 85 °C for 1 h and methyl vinyl ketone (0.53 mL, 4 eq.) added dropwise under a partial argon atmosphere. After letting react for 20 h, the mixture was cooled to room temperature and solvent removed under vacuum. The target material was purified by column chromatography (120 g SiO₂, 33% EtOAc in petroleum ether, R_{f} 0.3). The title compound was obtained as a colorless solid (1.45 g, 76% yield). Analytical data were in agreement with the literature.

The above results show that, desirably, Step F can be prepared in the same solvent mixture as Step B. Further, Step F can be efficiently performed utilizing the crude reaction product of Step B without substantial intervening purification or solvent removal.

### Example 5. Preparation of Compound BnO-IIIA-Bn (step D)

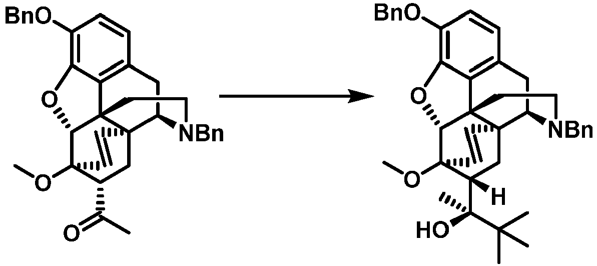

A 50 mL flask was charged with a solution of freshly prepared tert-butylmagnesium chloride (e.g., about 0.5 to 2 M, about 4-16 eq., preferably about 1.5 to 2M) in a mixture of THF and cyclohexane. To the flask was then added a solution of Compound BnO-II-Bn (0.5 g, 0.93 mmol) in dry toluene (8 mL). The reaction mixture was reacted overnight and then cooled in an ice-water bath and quenched by addition of 10% aqueous ammonium chloride (25 mL). The layers were separated and the aqueous layer was extracted with toluene (3x25 mL). The combined organic layers were washed with brine, dried with sodium sulfate, and concentrated. Purification by column chromatography (120 g SiO2, elution with 20% EtOAc in heptane) afforded Compound BnO-III-Bn as white solid (0.42 g, 83%). The spectral data were in agreement with literature data and of that reported in WO 2018/211331.

### Example 6. Preparation of Compound HO-IV-H (step C)

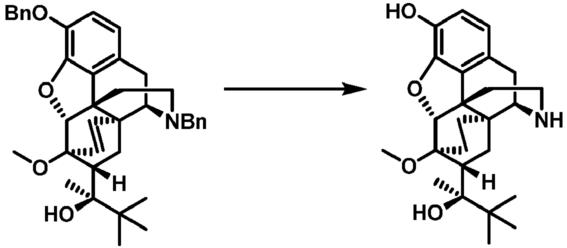

A vigorously stirred mixture of Compound BnO-III-Bn (355 mg, 0.6 mmol), and Pd/C (10%, 30 mg) in iPrOH (10 mL), water (0.2 mL), and acetic acid (0.1 mL) was hydrogenated at 60°C for 16 h under 1 atmosphere of hydrogen. IPC NMR showed that both benzyl groups were removed and the double bond was only partly reduced. The catalyst was refreshed and hydrogenation was continued at 80 °C for 60 h. ICP NMR showed no more double bond signals. The mixture was filtered over Celite. The filter was flushed with iPrOH and DCM. The filtrate was concentrated to give Compound HO-IV-H as acetate salt (300 mg, 100%). norbuprenorphine

HPLC-purity 89% at 215 nm.

MS (ES-API pos) m/z 414.3 (M+H).

¹H NMR (300 MHz, CDCl₃) δ [ppm] 7.64 (br s, 2H), 6.76 (d, *J* = 8.0 Hz, 1H), 6.49 (d, *J* = 8.1 Hz, 1H), 5.80 ( br s, 1H), 4.40 (s, 1H), 3.59 (d, *J* = 6.4 Hz, 1H), 3.51 (s, 3H), 3.35 - 3.25 (m, 2H), 3.04 (t, *J* = 13.5 Hz, 1H), 2.88 (dd, *J* = 19.2, 6.4 Hz, 1H), 2.75 (t, *J* = 13.5 Hz, 1H), 2.22 - 2.07 (m, 2H), 2.01 (s, 3H), 1.90-1.70 (m, 3H), 1.52 (dd, *J* = 13.1, 9.0 Hz, 1H), 1.33 (s, 3H), 1.18 (m, 1H), 1.03 (s, 9H), 0.76 (t, *J* = 12.3 Hz, 1H).

¹³C NMR (75 MHz, CDCl₃) δ [ppm] 145.91, 139.04, 129.99, 123.75, 120.29, 118.23, 95.53, 79.85, 79.62, 53.66, 52.69, 45.00, 42.97, 40.34, 34.40, 32.1, 31.8, 29.9, 29.1, 26.23, 22.9, 20.13, 17.8.

### Example 7. Preparation of buprenorphine (step A1)

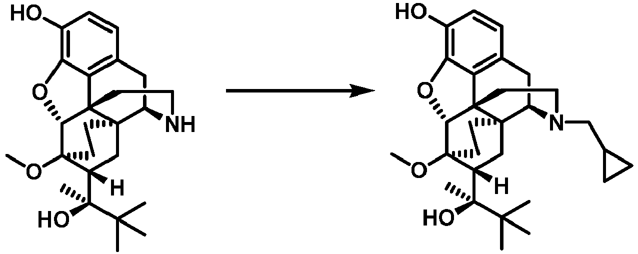

A 50 mL flask was charged with Compound HO-I-H (210 mg, 0.44 mmol), cyclopropane carboxaldehyde (80 µL, 1 mmol), dichloro(p-cymene)ruthenium(II) dimer (10 mg, 0.016 mmol), triethylamine (0.42 mL, 3.1 mmol), and acetonitrile (5 mL). The mixture was stirred under nitrogen at room temperature and formic acid (0.24 mL, 6.2 mmol) was added dropwise. The resulting mixture was heated at 60°C for 1 h. The mixture was cooled to room temperature and concentrated under vacuum. The residue was partitioned between toluene and 1 N aqueous NaOH. The aqueous layer was extracted twice with toluene. The combined organic layers were washed with brine, dried on sodium sulfate, and concentrated under vacuum to afford buprenorphine (160 mg, 78%).

HPLC-purity 85.6% at 215 nm.

MS and NMR data were in agreement with those obtained in previous examples.

## Claims

1. A method of preparing buprenorphine, or a salt thereof, from Compound HO-I-H, or a salt thereof: 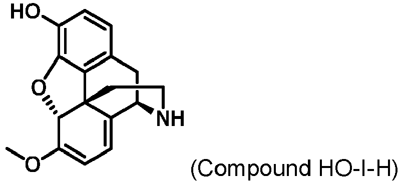 comprising:
(i)(F) in a first solvent system S1 comprising a polar protic solvent, reacting Compound HO-I-H with benzyl halide, benzyl sulfonate, or activated benzyl alcohol to provide Compound BnO-I-Bn: 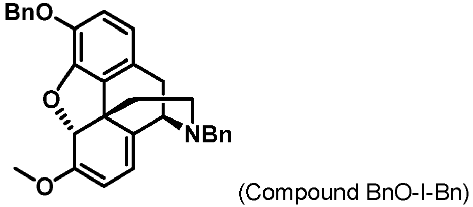
(ii)(B) in a second solvent system S2 comprising a polar protic solvent, reacting Compound BnO-I-Bn with methyl vinyl ketone to provide Compound BnO-II-Bn: 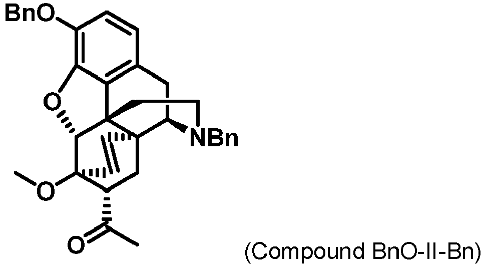
(iii)(D) in a third solvent system S3 comprising a nonpolar solvent, reacting Compound BnO-II-Bn with a tert-butylmagnesium compound to provide Compound BnO-IIIA-Bn: 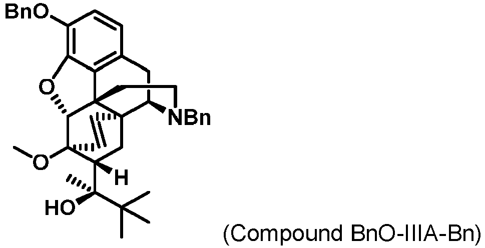
(iv)(C) reacting Compound BnO-IIIA-Bn with H₂ in the presence of a hydrogenation catalyst to provide a compound of Compound HO-IV-H: 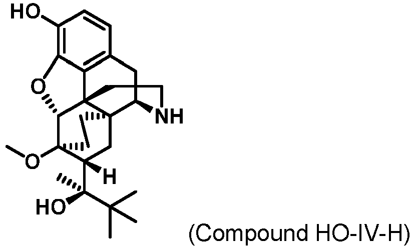
(v)(A1) reacting Compound HO-IV-H with cyclopropane carboxaldehyde followed by a hydride source; or
(v)(A2) reacting Compound HO-IV-H with cyclopropanecarboxylic acid halide followed by a reducing agent; or
(v)(A3) reacting Compound HO-IV-H with cyclopropylmethyl halide or activated cyclopropane methanol;
to provide buprenorphine.

2. The method according to claim 1, wherein S1 comprises at least one protic solvent having a dielectric constant of at least 12, or at least 14, or at least 16; optionally wherein S1 comprises at least 50 vol.%, or at least 75 vol.%, or at least 90 vol.% of the at least one protic solvent having a dielectric constant of at least 12.

3. The method according to claim 1, wherein S1 comprises at least one protic solvent having a polarity index of at least 3, or at least 3.5, or at least 4, optionally wherein S1 comprises at least 50 vol.%, at least 75 vol.%, or at least 90 vol.% of the protic solvent having a polarity index of at least 3.

4. The method according to any of the preceding claims, wherein S2 comprises at least one protic solvent having a dielectric constant of at least 12, or at least 14, or at least 16, optionally wherein S2 comprises at least 50 vol.%, at least 75 vol.%, or at least 90 vol.% of the protic solvent having a dielectric constant of at least 12.

5. The method according to any of claims 1-3, wherein S2 comprises at least one protic solvent having a polarity index of at least 3, or at least 3.5, or at least 4, optionally wherein S2 comprises at least 50 vol.%, or at least 75 vol.%, or at least 90 vol.% of the at least one protic solvent having a polarity index of at least 3.

6. The method according to any of the preceding claims, wherein S1 comprises isopropanol, optionally wherein S1 comprises 50-100 vol.% isopropanol and 0 to 50 vol.% water.

7. The method according to any of the preceding claims, wherein S2 comprises isopropanol, optionally wherein S2 comprises 50-100 vol.% isopropanol and 0 to 50 vol.% water.

8. The method according to any of the preceding claims, wherein step (ii)(B) is conducted in the presence of oxygen.

9. The method according to any of the preceding claims, wherein the methyl vinyl ketone of step (ii)(B) is added to a crude reaction product of step (i)(F), the crude reaction product comprising solvent S1 and Compound BnO-II-Bn.

10. The method according to any of the preceding claims, wherein S3 comprises at least one nonpolar solvent having a dielectric constant of at most 6, or at most 5, or at most 4, optionally wherein S3 further comprises at least 50 vol.%, or at least 75 vol.%, or at least 90 vol.% of the at least one nonpolar solvent having a dielectric constant of at most 6.

11. The method according to any of claims 1-9, wherein S3 comprises at least one nonpolar solvent having a polarity index of less than 3, or less than 2, or less than 1, optionally wherein S3 comprises at least 50 vol.%, at least 75 vol.%, or at least 90 vol.% of the at least one nonpolar solvent having a polarity index of less than 3.

12. The method according to any of the preceding claims, wherein S3 comprises less than about 10 vol.%, or less than about 5 vol.%, or less than about 2 vol.%, or less than about 1 vol.% of a total amount of solvents having a dielectric constant of greater than 6.

13. The method according to any of claims 1-11, wherein S3 comprises less than 10 vol.%, or less than 5 vol.%, or less than 2 vol.%, or less than 1 vol.% of total amount of solvents having a polarity index of 3 or greater.

14. The method according to any of the preceding claims, wherein S3 comprises 30-90 vol.% of one or more alkanes and/or cycloalkanes, optionally wherein the one or more alkanes and/or cycloalkanes comprises cyclohexane.

15. The method according to any of the preceding claims, wherein S3 comprises 10-50 vol.% toluene, 30-90 vol.% cyclohexane, and up to 30 vol.% tetrahydrofuran.

16. The method according to any of the preceding claims, wherein the tert-butylmagnesium compound comprises one or both of a *tert*-butylmagnesium halide and di-*tert*-butylmagnesium, optionally wherein the *tert*-butylmagnesium compound comprises a *tert*-butylmagnesium halide and di-*tert*-butylmagnesium.

## Patentansprüche

1. Verfahren zur Herstellung von Buprenorphin, oder einem Salz davon, aus der Verbindung HO-I-H, oder einem Salz davon: 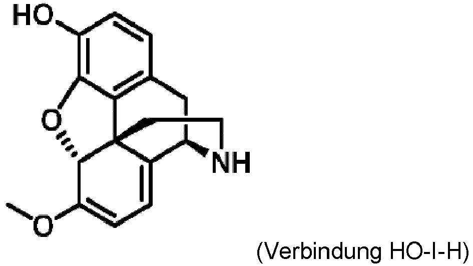 umfassend:
(i)(F) in einem ersten Lösungsmittelsystem S1, das ein polares protisches Lösungsmittel umfasst, Reagieren der Verbindung HO-I-H mit Benzylhalogenid, Benzolsulfonat oder aktiviertem Benzylalkohol, um die Verbindung BnO-I-Bn bereitzustellen: 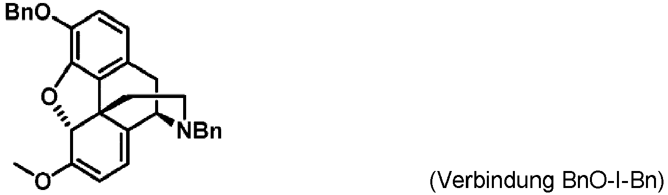
(il)(B) in einem zweiten Lösungsmittelsystem S2, das ein polares protisches Lösungsmittel umfasst, Reagieren der Verbindung BnO-I-Bn mit Methylvinylketon, um die Verbindung BnO-II-Bn bereitzustellen: 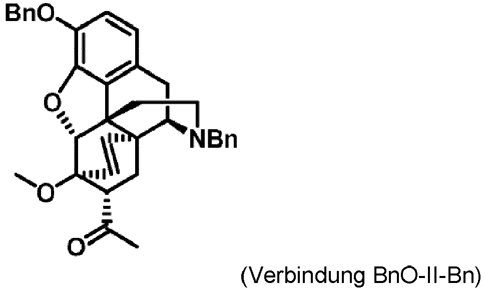
(iii)(D) in einem dritten Lösungsmittelsystem S3, das ein nicht polares Lösungsmittel umfasst, Reagieren der Verbindung BnO-ll-Bn mit einer tert-Butylmagnesiumverbindung, um die Verbindung BnO-IIIA-Bn bereitzustellen: 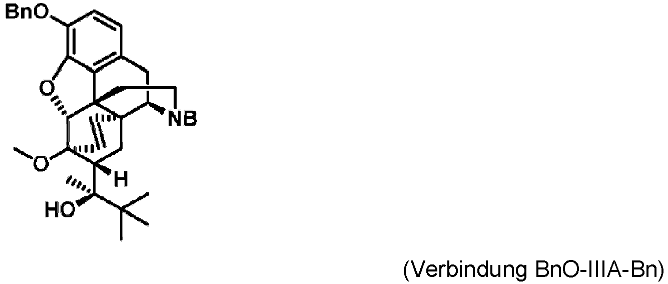
(iv)(C) Reagieren der Verbindung BnO-IIIA-Bn mit H₂ bei Vorhandensein eines Hydrierungskatalysators, um eine Verbindung der Verbindung HO-IV-H bereitzustellen: 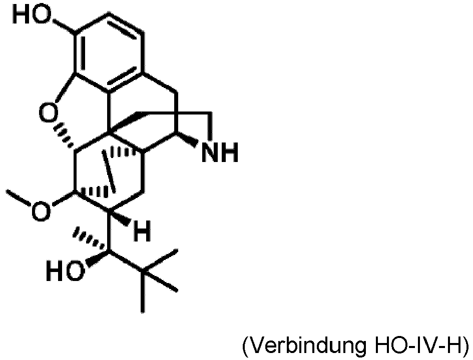
(v)(A1) Reagieren der Verbindung HO-IV-H mit Cyclopropancarboxaldehyd, gefolgt von einer Hybridquelle; oder
(v)(A2) Reagieren der Verbindung HO-IV-H mit Cyclopropancarbonsäurehalogenid, gefolgt von einem Reduktionsmittel; oder
(v)(A3) Reagieren der Verbindung HO-IV-H mit Cyclopropylmethylhalogenid oder aktiviertem Cyclopropanmethanol,
um Buprenorphin bereitzustellen.

2. Verfahren nach Anspruch 1, wobei S1 mindestens ein protisches Lösungsmittel mit einer Dielektrizitätskonstante von mindestens 12, mindestens 14 oder mindestens 16 umfasst; wobei S1 optional mindestens 50 Vol.-%, mindestens 75 Vol.-% oder mindestens 90 Vol.-% des mindestens einen protischen Lösungsmittels mit einer Dielektrizitätskonstante von mindestens 12 umfasst.

3. Verfahren nach Anspruch 1, wobei S1 mindestens ein protisches Lösungsmittel mit einem Polaritätsindex von mindestens 3, mindestens 3,5 oder mindestens 4 umfasst, wobei S1 optional mindestens 50 Vol.-%, mindestens 75 Vol.-% oder mindestens 90 Vol.-% des mindestens einen protischen Lösungsmittels mit einem Polaritätsindex von mindestens 3 umfasst.

4. Verfahren nach einem der vorhergehenden Ansprüche, wobei S2 mindestens ein protisches Lösungsmittel mit einer Dielektrizitätskonstante von mindestens 12, mindestens 14 oder mindestens 16 umfasst, wobei S2 optional mindestens 50 Vol.-%, oder mindestens 75 Vol.-% oder mindestens 90 Vol.-% des protischen Lösungsmittels mit einer Dielektrizitätskonstante von mindestens 12 umfasst.

5. Verfahren nach einem der Ansprüche 1-3, wobei S2 mindestens ein protisches Lösungsmittel mit einem Polaritätsindex von mindestens 3, mindestens 3,5 oder mindestens 4 umfasst, wobei S2 optional mindestens 50 Vol.-%, mindestens 75 Vol.-% oder mindestens 90 Vol.-% des mindestens einen protischen Lösungsmittels mit einem Polaritätsindex von mindestens 3 umfasst.

6. Verfahren nach einem der vorhergehenden Ansprüche, wobei S1 Isopropanol umfasst, wobei S1 optional 50-100 Vol.-% Isopropanol und 0 bis 50 Vol.-% Wasser umfasst.

7. Verfahren nach einem der vorhergehenden Ansprüche, wobei S2 Isopropanol umfasst, wobei S2 optional 50-100 Vol.-% Isopropanol und 0 bis 50 Vol.-% Wasser umfasst.

8. Verfahren nach einem der vorhergehenden Ansprüche, wobei der Schritt (ii)(B) bei Vorhandensein von Sauerstoff durchgeführt wird.

9. Verfahren nach einem der vorhergehenden Ansprüche, wobei das Methylvinylketon von Schritt (ii)(B) einem rohen Reaktionsprodukt von Schritt (i)(F) hinzugegeben wird, wobei das rohe Reaktionsprodukt das Lösungsmittel S1 und die Verbindung BnO-II-Bn umfasst.

10. Verfahren nach einem der vorhergehenden Ansprüche, wobei S3 mindestens ein nicht polares Lösungsmittel mit einer Dielektrizitätskonstante von maximal 6, maximal 5 oder maximal 4 umfasst, wobei S3 optional ferner mindestens 50 Vol.-%, oder mindestens 75 Vol.-% oder mindestens 90 Vol.-% des mindestens einen nicht polaren Lösungsmittels mit einer Dielektrizitätskonstante von maximal 6 umfasst.

11. Verfahren nach einem der Ansprüche 1-9, wobei S3 mindestens ein nicht polares Lösungsmittel mit einem Polaritätsindex von weniger als 3, weniger als 2 oder weniger als 1 umfasst, wobei S3 optional mindestens 50 Vol.-%, mindestens 75 Vol.-% oder mindestens 90 Vol.-% des mindestens einen nicht polaren Lösungsmittels mit einem Polaritätsindex von weniger als 3 umfasst.

12. Verfahren nach einem der vorhergehenden Ansprüche, wobei S3 weniger als etwa 10 Vol.-%, oder weniger als etwa 5 Vol.-%, oder weniger als etwa 2 Vol.-%, oder weniger als etwa 1 Vol.-% einer Gesamtmenge an Lösungsmitteln mit einer Dielektrizitätskonstante von mehr als 6 umfasst.

13. Verfahren nach einem der Ansprüche 1-11, wobei S3 weniger als etwa 10 Vol.-%, oder weniger als etwa 5 Vol.-%, oder weniger als etwa 2 Vol.-%, oder weniger als etwa 1 Vol.-% der Gesamtmenge an Lösungsmitteln mit einem Polaritätsindex von 3 oder größer umfasst.

14. Verfahren nach einem der vorhergehenden Ansprüche, wobei S3 30-90 Vol.-% von einem oder mehreren Alkanen und/oder Cycloalkanen umfasst, wobei optional das eine oder die mehreren Alkane und/oder Cycloalkane Cyclohexan umfassen.

15. Verfahren nach einem der vorhergehenden Ansprüche, wobei S3 10-50 Vol.-% Toluol, 30-90 Vol.-% Cyclohexan und bis zu 30 Vol.-% Tetrahydrofuran umfasst.

16. Verfahren nach einem der vorhergehenden Ansprüche, wobei die tert-Butylmagnesiumverbindung eins oder beide von einem tert-Butylmagnesiumhalogenid und di-tert-Butylmagnesium umfasst, wobei optional die tert-Butylmagnesiumverbindung ein tert-Butylmagnesiumhalogenid und di-tert-Butylmagnesium umfasst.

## Revendications

1. Procédé de préparation de buprénorphine, ou d'un sel de celle-ci, à partir du composé HO-I-H, ou d'un sel de celui-ci : 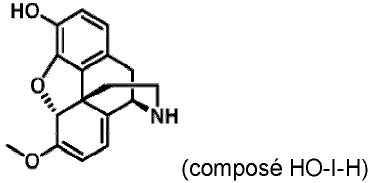 comprenant :
(i)(F) dans un premier système de solvants S1 comprenant un solvant protique polaire, la réaction du composé HO-I-H avec un halogénure de benzyle, du sulfonate de benzyle ou de l'alcool benzylique activé pour fournir le composé BnO-I-Bn : 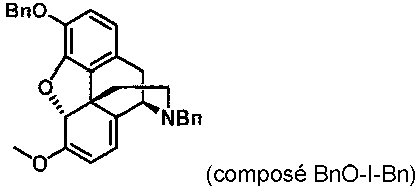
(ii)(B) dans un deuxième système de solvants S2 comprenant un solvant protique polaire, la réaction du composé BnO-I-Bn avec de la méthylvinylcétone pour fournir le composé BnO-II-Bn : 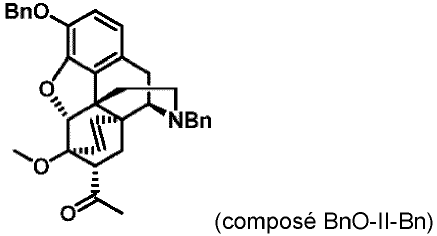
(iii)(D) dans un troisième système de solvants S3 comprenant un solvant non polaire, la réaction du composé BnO-II-Bn avec un composé tert-butylmagnésium pour fournir le composé BnO-IIIA-Bn : 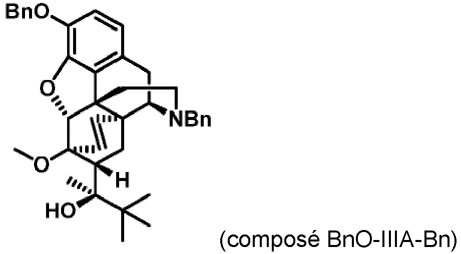
(iv)(C) la réaction du composé BnO-IIIA-Bn avec H₂ en présence d'un catalyseur d'hydrogénation pour fournir un composé du composé HO-IV-H : 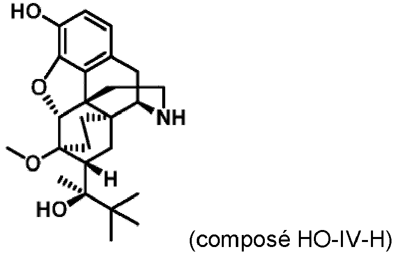
(v)(A1) la réaction du composé HO-IV-H avec du cyclopropanecarboxaldéhyde suivi d'une source d'hydrure ; ou
(v)(A2) la réaction du composé HO-IV-H avec un halogénure d'acide cyclopropane-carboxylique suivi d'un agent réducteur ; ou
(v)(A3) la réaction du composé HO-IV-H avec un halogénure de cyclopropylméthyle ou du cyclopropaneméthanol activé ;
pour fournir de la buprénorphine.

2. Procédé selon la revendication 1, dans lequel S1 comprend au moins un solvant protique ayant une constante diélectrique d'au moins 12, ou d'au moins 14, ou d'au moins 16 ; facultativement dans lequel S1 comprend au moins 50 % en volume, ou au moins 75 % en volume, ou au moins 90 % en volume de l'au moins un solvant protique ayant une constante diélectrique d'au moins 12.

3. Procédé selon la revendication 1, dans lequel S1 comprend au moins un solvant protique ayant un indice de polarité d'au moins 3, ou d'au moins 3,5, ou d'au moins 4, facultativement dans lequel S1 comprend au moins 50 % en volume, au moins 75 % en volume, ou au moins 90 % en volume du solvant protique ayant un indice de polarité d'au moins 3.

4. Procédé selon l'une quelconque des revendications précédentes, dans lequel S2 comprend au moins un solvant protique ayant une constante diélectrique d'au moins 12, ou d'au moins 14, ou d'au moins 16, facultativement dans lequel S2 comprend au moins 50 % en volume, au moins 75 % en volume, ou au moins 90 % en volume du solvant protique ayant une constante diélectrique d'au moins 12.

5. Procédé selon l'une quelconque des revendications 1 à 3, dans lequel S2 comprend au moins un solvant protique ayant un indice de polarité d'au moins 3, ou d'au moins 3,5, ou d'au moins 4, facultativement dans lequel S2 comprend au moins 50 % en volume, ou au moins 75 % en volume, ou au moins 90 % en volume de l'au moins un solvant protique ayant un indice de polarité d'au moins 3.

6. Procédé selon l'une quelconque des revendications précédentes, dans lequel S1 comprend de l'isopropanol, facultativement dans lequel S1 comprend 50 à 100 % en volume d'isopropanol et 0 à 50 % en volume d'eau.

7. Procédé selon l'une quelconque des revendications précédentes, dans lequel S2 comprend de l'isopropanol, facultativement dans lequel S2 comprend 50 à 100 % en volume d'isopropanol et 0 à 50 % en volume d'eau.

8. Procédé selon l'une quelconque des revendications précédentes, dans lequel l'étape (ii)(B) est conduite en présence d'oxygène.

9. Procédé selon l'une quelconque des revendications précédentes, dans lequel la méthylvinylcétone de l'étape (ii)(B) est ajoutée à un produit de réaction brut de l'étape (i)(F), le produit de réaction brut comprenant le solvant S1 et le composé BnO-II-Bn.

10. Procédé selon l'une quelconque des revendications précédentes, dans lequel S3 comprend au moins un solvant non polaire ayant une constante diélectrique d'au plus 6, ou d'au plus 5, ou d'au plus 4, facultativement dans lequel S3 comprend au moins 50 % en volume, ou au moins 75 % en volume, ou au moins 90 % en volume de l'au moins un solvant non polaire ayant une constante diélectrique d'au plus 6.

11. Procédé selon l'une quelconque des revendications 1 à 9, dans lequel S3 comprend au moins un solvant non polaire ayant un indice de polarité inférieur à 3, ou inférieur à 2, ou inférieur à 1, facultativement dans lequel S3 comprend au moins 50 % en volume, au moins 75 % en volume, ou au moins 90 % en volume de l'au moins un solvant non polaire ayant un indice de polarité inférieur à 3.

12. Procédé selon l'une quelconque des revendications précédentes, dans lequel S3 comprend moins d'environ 10 % en volume, ou moins d'environ 5 % en volume, ou moins d'environ 2 % en volume, ou moins d'environ 1 % en volume d'une quantité totale de solvants ayant une constante diélectrique supérieure à 6.

13. Procédé selon l'une quelconque des revendications 1 à 11, dans lequel S3 comprend moins de 10 % en volume, ou moins de 5 % en volume, ou moins de 2 % en volume, ou moins de 1 % en volume de la quantité totale de solvants ayant un indice de polarité de 3 ou plus.

14. Procédé selon l'une quelconque des revendications précédentes, dans lequel S3 comprend 30 à 90 % en volume d'un ou plusieurs alcanes et/ou cycloalcanes, facultativement dans lequel les un ou plusieurs alcanes et/ou cycloalcanes comprennent du cyclohexane.

15. Procédé selon l'une quelconque des revendications précédentes, dans lequel S3 comprend 10 à 50 % en volume de toluène, 30 à 90 % en volume de cyclohexane et jusqu'à 30 % en volume de tétrahydrofurane.

16. Procédé selon l'une quelconque des revendications précédentes, dans lequel le composé de *tert*-butylmagnésium comprend l'un ou les deux parmi un halogénure de *tert*-butylmagnésium et le di-*tert*-butylmagnésium, facultativement dans lequel le composé de *tert*-butylmagnésium comprend un halogénure de *tert*-butylmagnésium et du di-tert-butylmagnésium.
